(19) Europäisches Patentamt
European Patent Office
Office européen des brevets

(11) **EP 4 057 291 A1**

(12) **EUROPEAN PATENT APPLICATION**
published in accordance with Art. 153(4) EPC

(43) Date of publication:
**14.09.2022 Bulletin 2022/37**

(51) International Patent Classification (IPC):
***G16H 20/60*** (2018.01)   ***G06Q 90/00*** (2006.01)

(21) Application number: **20885470.3**

(22) Date of filing: **03.11.2020**

(86) International application number:
**PCT/RU2020/050312**

(87) International publication number:
**WO 2021/091430 (14.05.2021 Gazette 2021/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **04.11.2019  RU 2019135549**

(71) Applicant: **Atlas Biomed Group Limited
London EC4M 7AU (GB)**

(72) Inventors:
• **NIKOGOSOV, Dimitri
  Moscow,  121069 (RU)**

• **POPENKO, Anna Sergeevna
  Moscow,  121069 (RU)**
• **KARDAKOVA, Maria Yuryevna
  Moscow,  121069 (RU)**
• **LOSHKAREV, Robert Igorevich
  Moscow,  121069 (RU)**
• **MUSIENKO, Sergei Vladimirovich
  Moscow,  121069 (RU)**
• **PERFILYEV, Andrey Valentinovich
  Moscow,  121069 (RU)**

(74) Representative: **Papula Oy
P.O. Box 981
00101 Helsinki (FI)**

(54) **DIET TRACKING AND GENERATION OF A DIET QUALITY REPORT**

(57)   The present disclosure generally relates to microbiology and nutrition as well as to the use of computer engineering in microbiology and nutrition and specifically relates to methods and systems for tracking diet and forming conclusions on the diet quality and/or personalized nutrition recommendations. In this disclosure, from data on a user's intestinal microbiota, a list of recommended food products is generated for the user, where each food product has its own score obtained from a food product rating based on the user's intestinal microbiota condition; and then personal conclusions on the diet quality and recommendations for improving it are formed. The technical result is an increase in the quality and accuracy of forming personalized nutrition recommendations for the user.

Fig. 1

**Description**

FIELD OF THE INVENTION

The present disclosure generally relates to microbiology and nutrition as well as to the use of computer technology in microbiology and nutrition and specifically relates to methods and systems for tracking diet and forming conclusions on the diet quality and/or personalized nutrition recommendations.

Nowadays, scientists have proven that the microbiota of the human gastrointestinal tract has great effect on the host body through its immunity and metabolism. This relation is implemented through a number of substances secreted by the microbiota, including vitamins and short-chain fatty acids (SCFA). Dysbiosis leads to an imbalance in the interaction between the microbiota and the host body, contributing to the development of chronic inflammatory conditions. This along with other factors can lead to various serious diseases: type II diabetes mellitus, Crohn's disease, ulcerative colitis, obesity etc. Biomarkers of a number of diseases, including those listed above, were found in the microbiota - these are microorganisms increased or decreased abundance of which is significantly associated with certain diseases.

It is also known that the composition of the intestinal microbiota can be influenced through food or dietary supplements, such as probiotics, prebiotics by means of creating competitive advantages for certain bacteria. One of the variants of influence is an increase in the types and quantity of consumed dietary fibers, i.e. complex carbohydrates that are not digestible by humans. These substances are not digested by gastric acid and intestinal enzymes, and they are metabolized by intestinal microbiota bacteria.

The background art contains numerous sources of information indicating the importance of using probiotics (Isolauri et al. 2000; Kim et al. 2010; Singh et al. 2013) (live symbiotic microorganisms, which taken in adequate amounts, can have a beneficial effect on human health - both separately and in the composition of food products, e.g. fermented milk products), as well as a balanced diet rich in dietary fibers (acting as prebiotics - food ingredients not digested by humans, but contributing to the growth or increase in the activity of symbiotic microorganisms) to maintain a healthy microbiota in the human gastrointestinal tract and potentially prevent the development of symptoms of the above-mentioned diseases or improve them (Khaw and Barrett-Connor 1987; Ludwig et al. 1999; Anderson et al. 1987). However, clinical studies examining the effectiveness of probiotics and prebiotics in the prevention and treatment of these diseases have shown mixed results (Rondanelli et al. 2017; Makki et al. 2018). The studies show that not all people react equally to the same dietary supplements, and the reaction of the body depends on the initial composition of the microbiota in the gastrointestinal tract of the individual. For example, the same product can cause significant changes in the gastrointestinal microflora of some individuals and cause only small changes in others (Klimenko N. S., et al., Microbiome Responses to an Uncontrolled Short-Term Diet Intervention in the Frame of the Citizen Science Project. Nutrients. 2018 May 8;10(5)). Thus, despite the presence of general recommendations for a balanced diet for a wide range of population, there is a need for methods of developing personalized recommendations on nutrition and a list of wholesome food products for a particular individual, taking into account the composition of his/her gastrointestinal microbiota.

SUMMARY OF THE INVENTION

The technical task or technical problem solved in the present disclosure is forming ranked personalized nutrition recommendations for a user.

The technical result achieved in solving the above-mentioned technical problem is to improve the quality and accuracy of forming personalized nutrition recommendations for a user.

The above-mentioned technical result is achieved by tracking the user's diet as well as by scoring eaten dishes.

The above-mentioned technical result is achieved by implementing the method of tracking diet and forming conclusions on a user's diet quality and/or personalized nutrition recommendations for a user, executed by at least one processor and comprises obtaining data on a user's intestinal microbiota condition, containing at least one biomarker value; determining a value of at least one trait related to the user's intestinal microbiota condition, based on at least one biomarker value obtained; generating a list with at least one food product for the user, based on at least one biomarker value obtained and/or at least one trait value related to the user's intestinal microbiota condition and determined in the previous step; forming a personalized food product rating from the list generated in the previous step; obtaining data on at least one food product or dish of the user; determining a score for at least one food product or dish obtained in the previous step, based on the personalized food product rating formed earlier; forming at least one conclusion on the user's diet quality, based on the score previously obtained, and/or at least one recommendation for the user, based on the personalized food product rating.

In some embodiments, data on the user's intestinal microbiota condition are obtained by sequencing a biomaterial sample and/or because of a biochemical or microbiological analysis.

In some embodiments, the biomarker may be the relative or absolute abundance of microorganisms and/or their genes in the biological material.

In some embodiments, in forming a personalized food product rating, the rating value is calculated from the cumulative effect of this product on each trait associated with the user's intestinal microbiota condition; and the higher is the rating, the more useful is the product for the user's intestinal microbiota.

In some embodiments, a biomarker can be positively or negatively associated with a particular trait, depending on the biomarker effect on the trait value.

In some embodiments, a food product can be positively or negatively associated with a particular biomarker, depending on the food product effect on the biomarker value.

In some embodiments, the value of a trait related to the user's intestinal microbiota condition is evaluated in numerical and/or symbolic form.

In some embodiments, the trait related to the user's intestinal microbiota condition is the microbiota ability to synthesize metabolites by using the known metabolic pathways, or the microbiota ability to break down dietary fibers, or the glycemic response to a product or a dish, or the ability to break down gluten, or the ability to break down lactose, or microbiota barrier functions or a disease protection for which the connection with at least one microbiota biomarker or the probiotic level or another beneficial bacteria level is known.

In some embodiments, each trait is evaluated individually or grouped by a trait type with evaluating the entire group of traits in total.

In some embodiments, the initial level of the abundance of a biomarker in the user's intestinal microbiota, or the significance of the trait itself, or the number of biomarkers within one trait or group of traits influenced by a certain product are taken into account when forming a personalized food product rating.

In some embodiments, the rating for a food product is calculated as a weighted sum for all the user's traits when forming a personalized food product rating.

In some embodiments, a shortlist of recommended food products for a predetermined period of time is generated when forming a personalized food product rating.

In some embodiments, such a shortlist of food products is randomly generated from a complete list of food products that are positively recommended to the user, where the higher is the probability of a product getting shortlisted for a predetermined period of time, the higher is the rating of this product.

In some embodiments, the shortlist of food products is updated after each predetermined period of time in.

In some embodiments, a fixed number of items is allocated for each category of food products in the shortlist of recommended food products for a predetermined period of time.

In some embodiments, data on at least one food product or dish of the user is a list of food products or dish ingredients provided by the user, or an image of a dish or a food product, or an audio recording with a list of food products, or the composition of a dish and its cooking method.

In some embodiments, an image of a dish or a food product is recognized by an artificial neural network that receives an input of an image of a dish or a food product and generates an output of an N-dimensional vector of visual features of this image, and the visual information of the image analyzed is encoded in the vector.

BRIEF DESCRIPTION OF THE DRAWINGS

The features and advantages of the present disclosure will be evident from the following detailed description and attached drawings where:

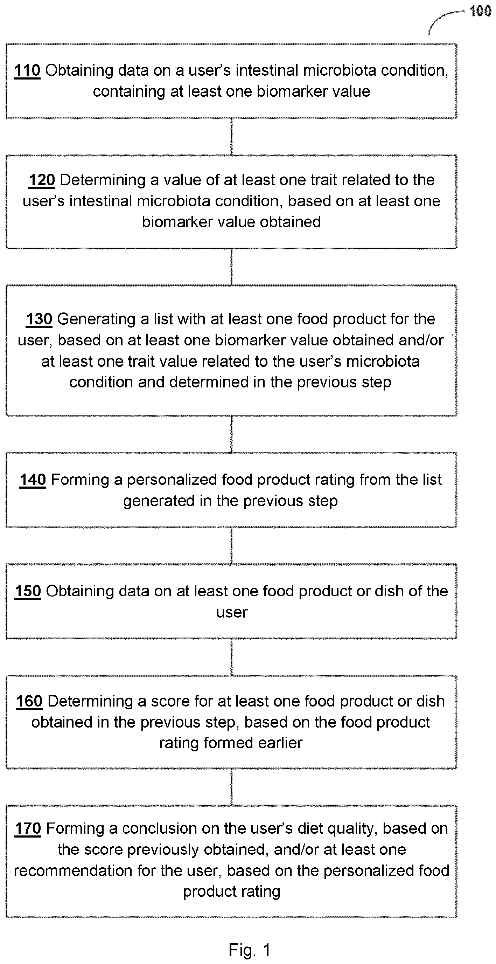

Fig. 1 shows an exemplary embodiment of the method of tracking diet and forming conclusions on a user's diet quality and/or personalized nutrition recommendations for a user in the form of a flow diagram.

Fig. 2 shows an exemplary embodiment of the graphical user interface of a mobile application, which displays the recognition of a set of ingredients in a dish image.

Fig. 3 shows an example of a user's rated dish and an explanation of how some food products in the dish were rated.

Fig. 4 shows an exemplary embodiment of the system of tracking diet and making conclusions on a user's diet quality and/or personalized nutrition recommendations for a user.

Fig. 5 shows an exemplary embodiment of generating a list for a new week (507) from a complete list of positively recommended food products (501).

DETAILED DESCRIPTION OF THE INVENTION

The present disclosure can be implemented on a computer or another data processing device in the form of an automated system or a computer-readable medium containing commands for the embodiment of the above-mentioned method.

The present disclosure can be implemented in the form of a distributed computer system, the components of which are cloud or local servers.

Some parts of this invention are presented in terms of algorithms and other representations of operations with data bits or binary digital signals in a computer memory. It is appreciated that definitions or conclusions mentioned in this description can be implemented with the use of artificial intelligence techniques. More specifically, the terms "processing," "calculation," "determination," "establishment," "analysis," "identification," "verification" etc., may relate to the operations and/or processes of a computer, computing platform, computer system or another electronic device that manipulates and/or transforms data represented as physical (electronic) quantities within the computer registers and/or memory devices into other data similarly represented as physical quantities within registers and/or storage devices of a computer or another storage medium that can store commands for executing the aforementioned methods, operations and/or processes.

The terms and their definitions used in the description of the invention will be considered in detail below.

In the present disclosure, a system means a computer system or an automated system (AS), an electronic computing machine (ECM), numeric control (NC), a programmable logic controller (PLC), a computer-assisted command system, a mobile device and any other devices capable of performing a specified, clearly defined sequence of calculation operations (actions, commands).

A command-processing device means an electronic unit or an integrated circuit (microprocessor) which performs computer-based commands (programs).

The command-processing device reads and executes computer-based commands (programs) from one or more data storage devices. A data storage device may represent hard disk drives (HDD), flash memory, memory cards, read-only memory (ROM), solid-state drives (SSD), optical disc drives (ODD), cloud storages etc.

A program is a sequence of commands to be executed by a computer control device or a command-processing device.

Microbiota is the totality of all microorganisms in a bacterial community.

Bacterial (taxonomic) composition of a microbiota sample is a list of all species (genera, taxa) identified in this sample during biochemical or microbiological analysis.

Bacterial compositions of microbiota samples can be analyzed at different taxonomic levels (species, genera, families etc.), so the description uses the term "taxon", which includes any of these levels.

The relative composition of a microbiota sample is the relative quantitative ratio of taxa or, in other words, the relative abundance of taxa in this sample. The relative abundance of microorganisms or their genes in a sample may be expressed in percents, portions, the number of reads or other units. For example, relative abundance may be given by a numeric value from 0 to 1 for each taxon in a sample, so that the total value for all taxa present in one sample is 1, and any other convenient scale may be used.

In some embodiments of the present disclosure, intestinal microbiota samples can be obtained by analyzing the user's samples of faeces. In alternative embodiment, microbiota samples can be obtained from analysis of any other biomaterial of the user, such as a biopsy, a smear, a swob, etc.

A biomarker is a measurable biological factor (for example, the relative abundance of a bacterial species or a bacterial genus) associated with the presence or absence of some condition in the host organism, for example, a certain disease.

Diet is the products consumed by a user. Tracking diet means monitoring a user's nutrition for a predetermined period of time, such as a day, a week, a month etc.

Nutrients are organic and inorganic substances that are part of food products and are used by the body to support its vital functions, i.e. proteins, fats, carbohydrates, dietary fibers, vitamins, minerals, short-chain fatty acids etc.

In the present disclosure, a nutrition recommendation means a recommendation on a product that influences the intestinal microbiota condition of a particular user. The recommendation is positive where the product improves a user's intestinal microbiota condition or maintains a healthy microbiota condition, and the product is recommended for use. A negative recommendation means a negative effect of the product on a user's intestinal microbiota condition, i.e. the product is not recommended for use.

The basic principles of healthy nutrition are increasing the dietary diversity, introducing various dietary fibers and other nutrients to the diet, consuming fermented milk products or other probiotics, consuming food products rich in vitamins and micronutrients, restricting or excluding certain food products from the diet.

Prebiotics are substances that are not utilized or not fully utilized by the human body, but are catabolized by intestinal microbes. These are polysaccharides (such as pectin, long-chain inulin, resistant starch) and oligosaccharides (fructooligosaccharides, galactooligosaccharides etc.). There are food products rich in a certain dietary fiber (for example, topinambur and chicory in the case of inulin). When processing prebiotics with intestinal bacteria, substances that have a beneficial effect on the human body - such as short-chain fatty acids (SCFA) - are secreted. The ability to process particular types of dietary fibers varies significantly among various taxa, and the breakdown of some complex branched polysaccharides occurs in several stages, with one of several cooperating bacterial species active in each stage. Thus, the ability of the individual intestinal community to break down different types of dietary fibers varies widely; that creates the basis for personalizing recommendations for the intake of prebiotics.

Probiotics are living bacteria that benefit the host; they can be consumed as isolated components or as part of food products (usually fermented milk products). Bifidobacterium (B. animalis, B. longum, B. bifidum species) and Lactobacillus (L. rhamnosus, L. casei, L. reuteri species) belong to the genera that include the largest number of used strains with probiotic properties.

Despite a general awareness of the healthy nutrition principles, the formation of proper dietary habits remains a critical task, especially among urban residents.

This invention allows a user to receive personalized nutrition recommendations based on data on his/her intestinal microbiota composition, track his/her diet with getting scores for eaten dishes and also diversify his/her diet thanks to an updated collection of recommended food products.

**Step 110:** obtaining data on a user's intestinal microbiota condition, containing at least one biomarker value.

A sample of biological material can be obtained using a collection kit, which may include a sample container having a process reagent component, and configured to receive a sample from a point of collection by a user, which can be remote. Additionally or alternatively, the collection kit can be provided directly through a device that is installed indoors or outdoors and is designated to facilitate receiving a sample from a user. In other embodiments, the collection kit can be submitted to a medical laboratory technician in a clinic or other medical institution, and earlier submitted to a user, for example, by courier. However, one or more user collection kits can be additionally or alternatively submitted to the unit of obtaining primary data by any other suitable way, for example, in frozen form in a sterile container.

Input samples of biological material can be represented by faeces samples, which can be processed, for example, in a laboratory and from which data on the composition of the intestinal microbiota are obtained by means of sequencing. The processing may include the stages of sample purification and total DNA extraction, including that of bacteria and archaea. Data on the composition of intestinal microbiota can be obtained, for example, as a result of sequencing microbial 16S rRNA gene sequences of the microbiota sample. In some embodiments, files of sequencing in FASTQ or FASTA format are obtained from the sequencer, one file per each sample. It is preferable to use amplicon sequencing, but whole genome (shotgun) sequencing may also be used without limitation.

In the case of analysis of the intestinal microbiota by using 16S rRNA sequencing after preprocessing, quantitative and qualitative taxonomic analysis of data is performed by determining to which known bacterium each read of 16S rRNA (or its fragment) belongs and how reads of unknown bacteria can be characterized. The search is performed with the use of reference-based search strategies. The taxonomic classification rests on the basic concept of operational taxonomic units (OTUs), i.e. determination of bacterial species based solely on its sequence of 16S rRNA gene. A set of reads of the 16S rRNA gene (or its region) is compared with the representative database of sequences of this gene. Each read is assigned to a taxonomic unit, with which it has a high degree of similarity. In case of several matches, it is possible to accidentally assign a read to one of these OTUs. In the database, each record is a representative sequence of the corresponding OTU obtained earlier as a result of cluster analysis. Whereas the similarity threshold can be varied, a similarity threshold of 97% is traditionally used in metagenomic studies as a heuristic evaluation of the degree of similarity in 16S rRNA within a single bacterial species. However, this value is not absolute: on the one hand, bacteria with very diverse sequences of this gene can occur within the same bacterial species, on the other hand, two different species can have identical sequences (for example, Escherichia and Shigella).

In some embodiments of the present disclosure, other two main strategies for OTU identification, known from the background art can be used: de novo search and a hybrid approach (combining elements of reference-based search and de novo search).

The sequences accumulated after 16S rRNA sequencing the microbiota are summarized in merged databases and are phylogenetically annotated. Among the most used databases in the background art can be used Greengenes (a database of whole sequences of the 16S rRNA gene), SILVA (includes sequences not only 16S, but also ITS, 18S, 23S/28S for eukaryotes), RDP (the annotation is less unified, but the capacity is higher than that of Greengenes) etc.

In one embodiment, processing a set of metagenomes in the format of 16S rRNA results in a table of relative abundance of the sample, which reflects the number of reads assigned to each operational taxonomic unit (OTU) from the database. Such a table of relative abundance may be rarified and/or normalized by any known method.

In some embodiments, the relative abundance is normalized. For this purpose, the number of reads successfully mapped against the reference database is summarized for each sample. The normalized abundance for each taxon is calculated as the number of reads assigned to this taxon for a given sample divided by the total amount of mapped reads for this sample and additionally multiplied by 100% in some embodiments. A normalized abundance table including the portion or percentage of reads assigned to each taxon from the database for each sample is compiled from obtained values of the normalized abundance. Normalizing is used for correct comparison of the abundance of the same biomarker or a certain taxonomic unit between different microbiota samples.

Further, low abundant taxa may be filtered out, for example, based on the following principle: taxa with abundance exceeding 0.2% of the total microbial abundance in at least 10% of the samples are retained.

**Step 120:** determining a value of at least one trait related to the user's intestinal microbiota condition, based on at least one biomarker value obtained.

Based on the obtained relative abundance of microorganisms and/or their genes in the intestinal microbiota, biomarkers (for example, taxa) associated with one or more traits that characterize the condition of the user's intestinal microbiota are identified. The value of the trait shows how the user's intestinal microbiota influences this trait. Knowledge and facts about the relations between a bacterial taxon and a trait, as well as between a bacterial taxon and a food product, are stored in a pre-constructed database that contains associations and relations between biomarkers and traits and/or food products.

In one of embodiment of the present disclosure, the traits may include:

- certain diseases for which a relation with at least one microbiota biomarker is known (the value of the trait indicates the level of protection against a particular disease based on the relative abundance of microorganisms or their genes);
- the level of probiotic and other beneficial bacteria (the relative abundance of beneficial bacteria is compared with the limits of normal in the population), the list of such is considered to be known;
- the microbiota ability to synthesize metabolites (vitamins, short-chain fatty acids (SCFA), amino acids, etc.) based on known metabolic pathways;
- the microbiota ability to break down dietary fibers.

In some embodiments, the glycemic response to certain food products or dishes for which a connection with at least one microbiota biomarker is known can be used as a trait. The glycemic response to certain food products (e.g., banana) or dishes (e.g., pasta) may be associated with the value of a particular biomarker of a user's intestinal microbiota. Taxa actively involved in the breakdown of dietary fibers may be an example of such biomarkers; the ability to quickly break down fibers can increase the rate of absorbing sugars and, consequently, the glycemic index of certain food products.

Other traits for which a relation with a particular biomarker is known can be used, such as the ability to break down gluten, ability to break down lactose, microbiota barrier functions, etc.

Each of the above-listed traits can be evaluated individually or grouped by trait type with evaluating the entire group of traits in total (merging in one trait). For example, the ability of microbiota to synthesize metabolites can be evaluated for each vitamin or SCFA for which there are data in the database of associations for the participation of a certain bacterium in the synthesis of this metabolite; in this case, the value for each metabolite may be considered a separate trait or all the values may be merged in one trait giving a resulting total value. The same is true for the rest groups of traits.

A biomarker can be positively or negatively associated with a particular trait depending on the bacterial taxon's effect on the trait value.

The value of a trait can be evaluated according to a certain numerical scale, for example, in points from 0 to 10, or from -1 (poor/low) to 1 (good/high) with 0 (neutral/normal), or categorically, or in any other way.

Some diseases may be associated with certain biomarkers obtained from the intestinal microbiota. Such associations can be obtained from scientific publications and studies. For example, the differences in the intestinal microbiota of people with type II diabetes mellitus and healthy people was studied: In the experimental group, Bacteroides genus bacteria prevailed and there were fewer Prevotella genus bacteria compared with the control sample set, while the abundance of Bifidobacterium spp and Bacteroides vulgatus was lower and the abundance of Clostridium leptum was higher than in the control group. In one embodiment, classifiers learned from study data, for example, using a random forest algorithm, can be used to identify such bacteria-disease associations. In this example, Bacteroides genus bacteria are positively associated with type II diabetes mellitus, this means that their high level worsens the trait of the disease protection, and the low level of such bacteria on the contrary improves the trait; Prevotella genus bacteria are negatively associated with type II diabetes mellitus, this means that their high level improves the disease protection, and the low level of such bacteria on the contrary worsens. The value of the trait of protection against type II diabetes mellitus consists of the effects of all taxa associated with this disease. A list of taxa that are recommended to be increased (if an inadequate relative abundance of a taxon negatively associated with a certain disease is identified in the microbiota) or to be decreased (if an increased relative abundance of a taxon positively associated with a certain disease is identified) can be obtained together with the trait value.

The value of a trait(s) responsible for the level of probiotic and other beneficial bacteria can be obtained by comparing the relative abundance of the known list of taxa in the user's intestinal microbiota with the limits of normal in the population for each taxon. For example, a relative abundance of 0.01% to 0.06% is considered the limits of normal in the population for Christensenella genus bacteria; respectively, 0.14% for Christensenella can be considered a high level of abundance for this taxon. The abundance of each beneficial taxon may be considered a separate trait, or the trait value may be calculated for the entire group of probiotics. A list of taxa, the relative abundance of which is recommended to be increased, can be obtained together with the trait value.

The value of a trait(s) responsible for the ability of microbiota to synthesize metabolites (for example, vitamins B1, B2, B3, B5, B6, B7, B9, K, butyric acid etc.) can be obtained as follows. In one embodiment, the relative abundance of microorganisms or their genes that make up the metabolic pathway for the synthesis of a certain vitamin, SCFA or other metabolite is determined in the intestinal microbiota composition. Usually, several taxa are involved in the synthesis of each metabolite. Their abundance is correlated with the average in the population based on which a conclusion is drawn about the microbiota ability to synthesize a particular metabolite. If the trait value for a certain metabolite is not high enough, it is possible to obtain a list of taxa, the relative abundance of which is recommended to be increased in order to improve the ability to synthesize this metabolite.

The value of a trait responsible for the microbiota ability to break down dietary fibers can be obtained by determining the breakdown potential for each dietary fiber type from a predetermined set. In one embodiment, the total relative abundance of taxa involved in the digestion of a particular dietary fiber can be evaluated and compared with the average in the population. The breakdown potential of each fiber can be considered a separate trait, or the trait value can be calculated for the entire group of the dietary fibers. A list of taxa, the relative abundance of which is recommended to be increased (if the breakdown potential of a particular fiber is low, then the abundance of the associated taxa can be considered reduced), can be obtained together with the trait value.

A database with associations that includes information on the known relations between bacterial taxa and traits characterizing the user's intestinal microbiota condition can be pre-formed. Additionally, the database with associations may include information on the normal or medium relative abundance of a taxon in the population or other limit values that allow determining the value of a particular trait. In the illustrative embodiment, the database with associations is implemented in the form of one or more databases (with the use of tables in this example), and a separate database can be constructed for each trait or type of relations; however, it will be obvious to a person skilled in the art that the database of associations can be implemented in other ways, which does not affect the essence of the invention.

In addition, the database with associations may contain information on the effect of various food products on bacterial taxa, the content of dietary fibers in certain products etc.

The database of associations for food products can be formed and updated using search and computer-aided text analysis algorithms together with manual filling based on the information on food products, the intake of which is positively or negatively associated with a particular taxon typical of the intestinal microbiota. The database with associations for food products may contain product-taxon relations for food products that influence a certain bacterial taxon and contain information on the effect type (positive, if the food product increases the relative abundance of this taxon, or negative, if it contributes to a decrease in the relative abundance). The database with associations for food products may also contain similar fiber-taxon relations for dietary fibers that influence certain taxa as well as information on the availability of dietary fibers in food products.

Examples of data fragments from the database of product-biomarker associations may be formed as follows:

| Biomarker | Food product | Food product effect on biomarker |
|---|---|---|
| Bifidobacterium | Yogurt | Increases |
| Staphylococcus | Oregano | Decreases |
| Bifidobacterium | Chicory | Increases |
| Lactobacillus | Chicory | Increases |
| Megasphaera | Chicory | Increases |
| Cam pylobacter | Chicory | Decreases |
| Brachyspira | Chicory | Decreases |

Examples of data fragments from the database of dietary fiber-biomarker associations may be formed as follows:

| Biomarker | Dietary fiber | Dietary fiber effect on biomarker |
|---|---|---|
| Alistipes | Inulin | Decreases |

(continued)

| Biomarker | Dietary fiber | Dietary fiber effect on biomarker |
|---|---|---|
| Prevotella | Inulin | Increases |
| Enterococcus | Inulin | Increases |
| Lactobacillus | Inulin | Increases |
| Bacteroides | Inulin | Increases |
| Bilophila | Inulin | Decreases |
| Faecalibacterium | Inulin | Increases |
| Bifidobacterium | Inulin | Increases |

**Step 130:** generating a list with at least one food product for the user, based on at least one biomarker value obtained and/or at least one trait value related to the user's intestinal microbiota condition and determined in the previous step.

After analyzing the data on the composition of the intestinal microbiota and determining the trait values, the user can access a list of recommended food products that contribute to improving the state of traits and/or contain a certain set of dietary fibers. This list is generated from the data on the relative abundance in the intestinal microbiota of biomarkers associated with a healthy microbiota, the proportion of which should be maintained or increased, as well as biomarkers associated with an unhealthy microbiota, the proportion of which should be decreased.

Recommendations for the user offer to increase the relative abundance of bacteria that are negatively associated with a particular disease, have a low (non-zero) or normal abundance and are not positively associated with other diseases as well as to decrease the relative abundance of bacteria that are positively associated with the disease. Similarly, recommendations are formed in regard to other traits, based on the list of taxa, the relative abundance of which was not high enough at the stage of calculating the trait value.

In one embodiment, an individual personalized list of recommended products may be generated as follows:

- for each disease or trait with a low or medium level of disease protection (trait value), taxa negatively associated with the disease and having a low (non-zero) or normal relative abundance of bacteria are selected, and bacteria positively associated with the disease and having a high relative abundance are also selected;
- taxa with a low (non-zero) relative abundance are selected from probiotic and other beneficial bacteria;
- associated taxa with a low (non-zero) relative abundance are selected for dietary fibers with a low or medium breakdown potential;
- taxa with a low (non-zero) relative abundance, which are typically involved in the synthesis of a given metabolite, are selected for metabolites with a low level of synthesis.

Further, food products associated with the selected taxa that are recommended can be found in the association database for food products. In one embodiment, if a product affects the user's traits (for example, it increases the abundance of a taxon recommended to be decreased), then it is also included in the recommendation list with a negative recommendation.

Thus, the list of the recommended food products includes all products that are associated with at least one trait and affect the condition of the user's intestinal microbiota. Additionally, some products may be excluded from this list - for example, due to allergy, intolerance - based on the results of a genetic test, diagnosis, Rome IV criteria etc. The user can also mark products to be excluded (for example, by preliminary questioning or by marking in the already generated list of recommended food products.)

If there are few recommended food products based on the traits of a particular user (for example, the composition of a healthy intestinal microbiota may have short list of traits to improve), then the list of recommended food products may be supplemented by supporting recommendations for probiotic and other beneficial bacteria from the system or by dietitian, for example, via a messenger or a chat bot.

Data that are a set of offered food products in a data storage may be accessed through a database management system (DBMS), which may contain one or more programs that control the organization, storage, management of, and search for data in the database. An embodiment of the present disclosure by using a cloud storage, e.g. Google Sheets, is also possible. The DBMS can receive requests for access to information in the database, which are generated, for example, in a mobile application based on its nutrition recommendations, and can execute operations necessary to ensure this access. Access used here may include data reading, data recording, data deleting, data updating, a combination of two or more of the above-mentioned steps, etc.

The database stored in the data storage may be a relational database, an object-oriented database, a hierarchical database, a network database, other types of databases, some combination or extension of the above-mentioned, etc. Data, which are a set of food products offered and stored in the database, may be organized in the form of tables, records, objects, other data structures etc. The data may also be stored in special database files, special hard drive partitions, HTML files, XML files, spreadsheets, flat files, document files, configuration files, other files etc. The database can refer to a read-only dataset or read a dataset and record into it.

When describing the aspects of this invention, sometimes terminology associated with relational databases is used here for the sake of simplicity. However, despite the use of terminology associated with relational databases herein, the presented ideas can be applied to other types of databases, including the above-mentioned.

A source(s) of data replenishment (enrichment) may include any other subject that is capable of supplying data. For example, the source(s) of data replenishment may include a service that receives data (e.g. a country code) and provides data (e.g. information on new dishes or food products of that country) in response to this. Each source of data replenishment can be implemented through a process executed by one or more computers.

In some embodiments of the present disclosure, the recommendation may be presented on the display of the user's mobile device or a personal computer, e.g. in a mobile application or on a website.

**Step 140:** forming a personalized food product rating from the list generated in the previous step.

In an alternative embodiment, the rating can be calculated for all food products available in the database.

For each product, the rating value is calculated, which will result from the effect of this product on each trait (the effect can be negative or positive); and the higher is the rating, the more useful is the product for the user's intestinal microbiota. If the rating is negative, then the recommendation for this food product is also negative, i.e. it is recommended to abandon the intake of this food product. If a food product does not influence the microbiota, its rating is zero.

It is worth noting that if the effect of a certain food product on a taxon is negative, but this taxon is recommended to be decreased due to a certain trait, then the effect of the food product on this trait will be positive. Similarly, if this taxon is recommended to be decreased due to a certain trait and the food product positively influences this trait, then the effect of the food product on this trait will be negative.

Various methods and formulas may be used to calculate the food product rating without limitation, while the method of calculation can take into account not only the type of effect on a particular trait (positive or negative), but also the initial level of taxon abundance in the user's intestinal microbiota, significance of the trait, number of taxa within one trait or group of traits influenced by this food product, as well as other parameters.

For example, the rating $w$ for a food product can be calculated as a weighted sum for all traits of the user $w = \Sigma_i p_i l_i k_i$, where $p_i$ is the type of effect on a particular trait (for example, -1 if the food product influences the trait negatively, 1 if the food product influences the trait positively, 0 if the food product does not influence); $l_i$ is the factor that takes into account the initial level of taxon abundance influenced by the food product within the framework of this trait (for example, corresponding factors for a high, normal or low level); $k_i$ is the correction factor that takes into account the significance of the trait itself (for example, 0.5 for effect on the trait from a group of metabolites, 1 for a disease, 2 for probiotics). Correction factors are selected in advance and allow adjusting the contribution of each trait; higher factors may also be set, if the trait value was calculated as common to the entire group of traits. The formula offered may contain an additional term or factor taking into account the number of taxa within the framework of this trait, which are positively or negatively influenced by the food product.

The rating of food products calculated in this way represents the benefit degree of a given food product for the user's intestinal microbiota and is personalized. Food products with a positive rating are regarded as recommended for use.

For each food product, reference information may be additionally stored and may include details on the food product effect on the user's intestinal microbiota and traits, general information on the benefit or harm of this food product, content of micronutrients, fibers, nutritional value, recommended daily intake, association with diseases or immunity, recommendations on the cooking method, interesting facts etc. For example, reference information may be available when viewing a product card (a separate panel or page); alternatively, it may be displayed directly in the list of recommended products.

In some embodiments of the present disclosure, a shortlist of recommended food products for a predetermined period of time is generated.

A predetermined period of time may be set, for example, in one week, day, month, quarter, half-year etc., without limitation. To understand the essence of the invention, an embodiment where the predetermined period of time is set to one week is described further.

Such a list contains a fixed number of products (e.g. 10), and the list is generated anew each week. As the list of recommended food products may contain a rather long inventory of items, the present disclosure offers to make a selection of several recommended food products that will be regularly updated, for example, once a week. This allows gradually familiarizing the user to the food products that are beneficial to be introduced to his/her diet.

A list for a week is randomly generated from a complete list of food products positively recommended to the user, where the higher is the rating of this food product, the higher is the probability of a food product getting into a list for a week. The list for a week is intended to encourage the user to try all the food products from this list for a week within the week. Instead of a week, any other fixed period of time may be used without limitation. In one embodiment, a list for a week is generated from a complete list of recommended food products in the way described below.

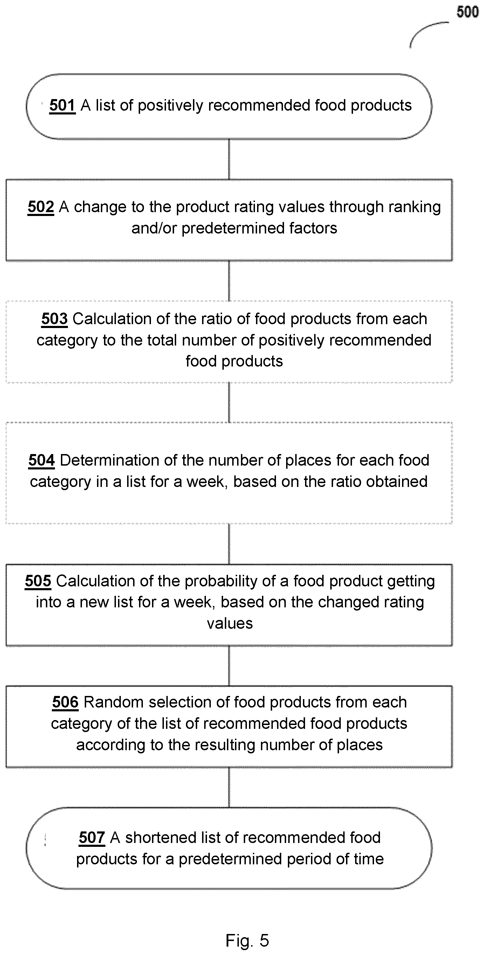

In one embodiment, to form a list for a new week (507), a complete list of positively recommended food products (501) can be ranked by rating value, as shown in Fig. 5. Food products displayed in the list for the current week can be sent to the end of the ranked list, regardless of their rating. Then the list can be divided into groups and the rating value of food products from different groups can be multiplied by predetermined factors in order to increase the difference between these values with new resulting values w' (502) and to influence the probability distribution. The probability that a product will be listed for a new week is proportional to the resulting values w'. For example, the list of the recommended food products may be divided into three equal groups (if the number of the list items is not a multiple of three, then there will be one more product in a group(s), e.g. in a group with food products of a higher rating.)

In an alternative embodiment, the probability that a product will be listed for a new week is proportional to personal the food product rating, where reduction factors (502) may be used to rate the food products from the current list for a week in order to reduce the recurrence rate.

In order to diversify a user's diet, when making a list for a week, additional conditions can be used so that the list contains food products from different categories, if such a category is present in a complete list of recommended food products. Examples of possible categories of food products are vegetables, fruits and berries, dairy products, cereals, beans, etc.

In one embodiment, the percentage or portion of food products from each category (503) present in a complete list of recommendations (501) is calculated, then a fixed number of items for each category (504) is allocated in a list for a week in accordance with their percentage or portion in a general list, but at least one item for each category. If any category is represented by a small number of recommended food products or at least one product, then at least one item in a list for a week will be allocated for it through a decrease in the number of items in the most represented category. If any category is not represented in a complete list of recommended food products, then items in a list for a week are not allocated for that category.

For example, to calculate the probability (505) of product selection, the following formula may be used:

$$p_i = \frac{w_i'}{\Sigma w_i'}$$

, where summing the rating values in the denominator is done for all food products from a list of positively recommended ones or for all food products from this list within each category separately.

A list for a week (507) is generated by randomly selecting N products from each category (506), where N is the number of allocated items, and the probability for the product to be selected corresponds to the probability previously calculated $p_i$ (502).

If a user selects a food product present in the current list for a week as excluded from his/her diet via a mobile application or a website, then it can be automatically excluded from the list for a week and replaced with another product.

In some embodiments of the present disclosure, lists of recommended or non-recommended food products are sent to a user's mobile device via any acceptable wireless technology, such as IEEE 802.11 standard, Bluetooth, UWB, Worldwide Interoperability for Microwave Access (WiMax) protocols or cellular protocols, e.g. General Packet Radio Service (GPRS).

**Step 150:** obtaining data on at least one food product or dish of the user.

A user can track his/her diet by using photographs or images or audio recordings of dishes eaten or a list of products provided by the user. An individual score that represents the general benefit for the user and the diversity of his/her diet is set for each dish or food product.

In some embodiments, data on a user's product eaten or to be eaten represent a user-entered list of food products or dish ingredients, an image of a dish or a food product (which is then recognized), an audio recording with a list of products (which is then recognized), a description of a food product with its weight, calories, micronutrients, manufacturer etc., for example, in doc, xls, pdf format, without limitation. It is obviously to a person skilled in the art that data on a food product may be represented in any known data format to be recognized.

An embodiment will be described in detail below, according to which data on a user's product or dish are represented in the form of an image.

For example, an image of a dish may be a photograph of any food and/or drink as well as a particular food product. A photograph or an image of food can be transmitted to the server via any user mobile device equipped with a photo- or video camera. The image of food can be uploaded via an application or a website, sent as a message via messengers, including chat bots, via mobile communication or in any other accessible way via any data communication channel.

Then, food products that make up the dish are identified based on at least one image obtained at the previous step and/or a food product itself is identified.

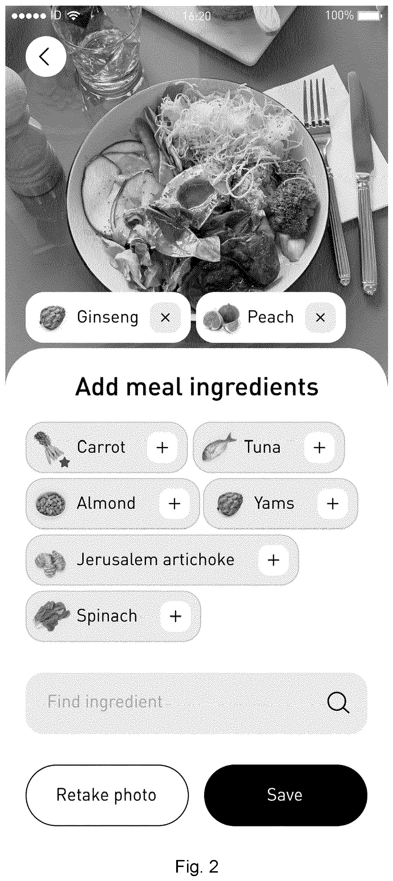

To identify food products present in the image, for example, a set of ingredients in the dish image may be recognized, as shown in Fig. 2. The user can mark food products present in the dish and in the list recognized by the system and/or enter other ingredients by searching by food product name. In some embodiments of the present disclosure, the list of recognized food products (ingredients) can be further adjusted by the user. If no food product was detected during the image recognition/analysis, food products can be manually entered/marked with the use of a product database, which may be stored, for example, in a database of associations.

In one embodiment, a list of food products in the dish image can be recognized as follows. For image analysis, a deep convolutional artificial neural network is used. The network receives an input of an image of a dish or a food product and generates an output of an N-dimensional vector of visual features of this image, and the visual information of the image analyzed is encoded in this vector. The resulting vector is then fed to the input of a decoder artificial neural network, which is, for example, a multilayer perceptron, RNN or Transformer decoder, without limitation. The decoder output is a set of vectors encoding certain ingredients of the dish. Alternatively, the output may be a probability vector for the entire set of analyzed ingredients, and the result of image recognition will be a set of ingredients that most closely matches the vector of the visual image features in terms of internal weights of the neural network.

In some embodiments, a convolutional neural network (CNN) is used in conjunction with a recurrent neural network (RNN).

In some embodiments of the present disclosure, other methods, such as Bayesian networks, multilayer neural networks, hidden Markov models, decision trees, SVM, kNN and more, may be used to recognize and classify food products.

Artificial neural networks are learned on a data set in the public domain, e.g. Recipe1M+, for which all dish ingredients have previously been filtered and marked in a semi-automatic mode. Moreover, the marking of dish ingredients may be a list of products corresponding to the image and may not require allocating of individual food products in the image. For example, a list of possible ingredients can be obtained by automated data collection (parsing) from websites with recipes. Complex ingredients of a dish are preliminarily decomposed into simpler components to reduce the dimensionality of the output vector of the neural network as well as to obtain a more relevant list of ingredients at the output (for example, "cottage cheese in cream" can be decomposed into "cottage cheese" and "cream" as only individual components are important for effect on the intestinal microbiota).

Further, each food product can be evaluated in terms of its benefits to a user's body and/or intestinal microbiota. Information on the nutrient content is used to evaluate the benefit of food products. Such information may include product-nutrient relations and the nutrient amount (e.g. the number of grams of a nutrient per 100 grams of a food product) and be stored in a product database, e.g. in a database of associations.

**Step 160:** determining a score for at least one food product or dish obtained in the previous step, based on the personalized food product rating formed earlier.

The score given to a food product may take into account various criteria related to the benefit of this product, such as the rating of this product in lists of recommended or non-recommended food products, presence of dietary fibers, macro- and micronutrients with their amount, variety or lack of variety in the diet, food product presence in a list for a week, food product fermentation, cooking method etc. If the product is absent in lists of recommended and non-recommended food products, its rating may be considered as zero.

Evaluation by some criteria may reduce the final product score (to impose a penalty) that is equivalent to scoring negative points.

In one embodiment, the score for a product can be calculated as the sum of the intermediate points for each of the predetermined criteria, and the number of points is prespecified.

An example of a calculation by criteria may be shown as follows:

- add points for a various diet when a given food product is first eaten in a week;
- impose a penalty for a dreary diet (deduct points) when a food product is eaten more than a determined number of times per week (for example, more than 4 times in the current week), this number may be the same or may be set separately for each product or product category;
- add points for the presence of a vitamin or other nutrient in a food product, the number of points may be the same for all nutrients or may differ, while an increased number of points may be given for high-nutrient food products;
- add points for the presence of dietary fibers in a food product, while an increased number of points may be given for high-fiber food products;
- impose a penalty for a high content of sugars;
- impose a penalty for the presence of saturated fats;
- impose a penalty for alcohol;
- add points for ingredient fermentation;
- add points for the presence of a food product in a list for a week;

- take into account the food product rating in the list of recommendations (add a score equal to the rating or to the rating multiplied by a predetermined factor).

Points/penalty for each of the criteria may be given for the presence of a certain nutrient in a food product (for example, 5 points are given for the presence of vitamin C in a food product) or when the nutrient amount in this product exceeds a certain value (for example, 5 points are given if the amount of vitamin C in a food product is greater than or equal to 12 milligrams per 100 grams of the food product; 10 points are given if its amount is greater than or equal to 24 milligrams per 100 grams of the food product).

Additionally, other criteria may be used to evaluate a product, taking into account its cooking method, the use of spices, serving size, energy value etc.

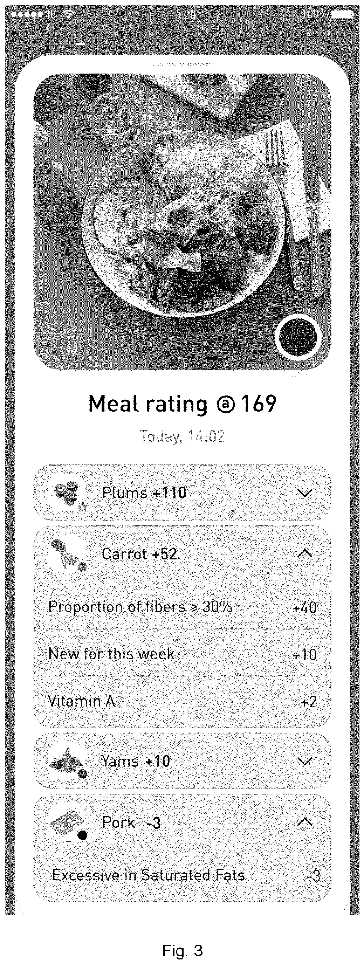

Fig. 3 depicts an example of a user's rated dish and an explanation of how some food products in the dish were rated.

The proposed method of evaluating food products considers not only general criteria of product benefits, but also individual needs of the user, the effect of the food product on the intestinal microbiota of this user.

In some embodiments of the present disclosure, the score is obtained based on calculations for the whole dish.

The final score given to a dish may be obtained by combining points for its separate ingredients (for example, by calculating the arithmetic mean, summing up etc.), i.e. points given to each food product from the dish composition. In some embodiments of the present disclosure, the final score may be normalized (for example, with values from 0 to 10).

Thus, in response to a dish image, which may be obtained at step 150, the user receives:

- an individual score of the dish benefits to the user's body and/or intestinal microbiota,
- an explanation of how this score has been determined, i.e. evaluation of each food product and/or criterion,
- information on each food product in the dish regarding the effect of this product on the user's traits.

In addition to general information on healthy nutrition, the proposed method allows the user to receive personalized information on the benefit of each eaten dish and/or food product, including information on how each product affects his/her intestinal microbiota.

**Step 170:** forming a conclusion on the user's diet quality, based on the score previously obtained, and/or at least one recommendation for the user, based on the personalized food product rating of the user.

A conclusion on a user's diet quality includes information on whether a food product or a dish, details about which are entered by the user, for example, in a mobile application or on a website, is healthy to the user's intestinal microbiota, and this conclusion is based on the number of points assigned to the product or dish at step 160 earlier. The number of points assigned may be based on the content of nutrients in the food product or dish, which influence the microbiota condition, the presence of fermentation products in the food product or dish, the presence of microorganisms in the food product, the presence of chemicals formed during the heat treatment of the food product, the presence of preservatives, stabilizers, coloring agents, flavors or other chemicals associated with a change in the condition of the user's intestinal microbiota. The conclusion is issued in the form of a text, an image or in audio format, for example, via a mobile application or on a website.

A nutrition recommendation for a user is a call to take actions aimed at changing the nature of the user's nutrition. They may include information on changes in the number of specific food products or dishes in the diet, on the number of probiotics and prebiotics consumed, dietary supplements, including vitamin and/or mineral and complex dietary supplements with other nutrients (e.g. fatty acids). It may also be a recommendation to change the number of meals, the volume of meal (serving), the time of meal. It may also be a recommendation to seek a medical advice from a medical specialist, nutritionist and/or dietitian. It may also be a request to enter additional data on the user, such as weight, height, diseases, additional genetic data etc. into the system, for example, via a mobile application. Recommendations are issued in the form of a text, an image or in audio format, for example, via a mobile application or on a website.

In addition to the score of the current dish, a user can access information on previously eaten dishes and/or uploaded images. An inventory of food products from the list for the current week is also displayed, while the listed products that the user has already consumed during the week visually differ from those that the user has not eaten even once during the current week. The user may have access to the summary of the results for the current week, day, month as well as past weeks, days, months or other periods of time. The summary may include the total number of points given, the total effect on the user's traits, the display of the complete diet (list of food products eaten and the number of times of consumption) for a specified period, etc.

The total effect on the user's traits may be displayed as follows. For each of the traits that require improvement according to the results of the microbiota analysis (i.e. those traits for which the nutrition recommendations are provided), the dynamics is shown for this trait for a specified period of time (e.g. a week); the dynamics may be shown in the categories "good"/"neutral"/"bad", with use of color coding, pictograms or other means; it informs the user how his/her diet for a given period of time has influenced this trait in total. For this purpose, the following principle may be used, for example, based on the final result for the week: the weekly diet had a "good" effect on a given trait if the difference between the number of dishes with a positive effect and the number of dishes with a negative effect exceeds the predetermined value; the weekly diet had a "bad" effect on a given trait if the number of dishes with a positive effect turned out to be less than the number of dishes with a negative effect by a predetermined value; in all other cases, the effect of the diet will be considered "neutral".

In some embodiments, additional points may be given depending on the weekly results; for example, if a user tried all food products from a list for a week, ate more than a certain number of different food products and/or different sources of dietary fibers during the week, etc. Comparison with the results of the last week, comparison with the score for the last week, day or month, comparison with the results of other users, etc. is also possible. A user is enabled to scroll weeks/days/months and go to the record card of a selected week/day/month, where all data are uploaded for the period specified.

The formation of healthy nutrition habits will improve the overall health of users and reduce the risks of diseases.

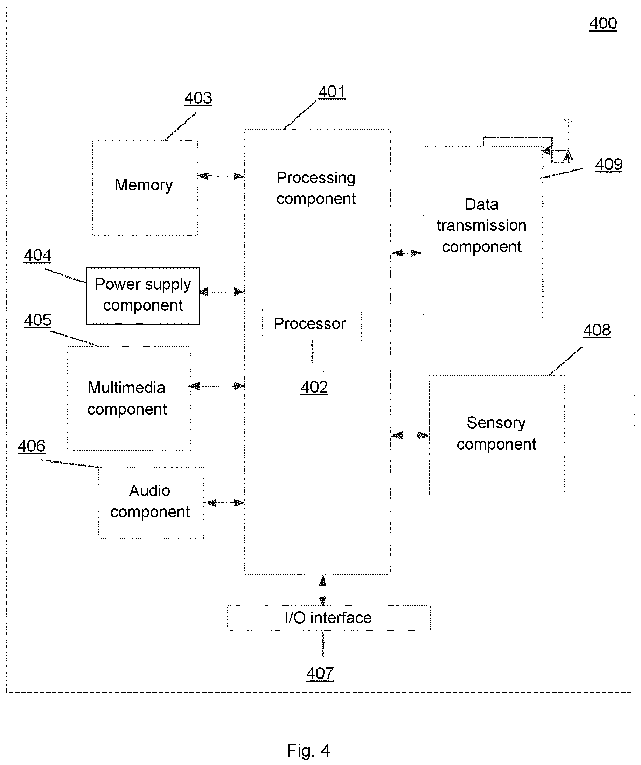

With reference to Fig. 4, the present disclosure can be implemented as a computing system **400** of tracking diet and forming conclusions on a user's diet quality and/or personalized nutrition recommendations for a user, which includes one or more of the following components:

- a processing component **401** including at least one processor **402,**
- a memory **403,**
- a multimedia component **405,**
- an audio component **406,**
- an input/output interface (I/O) **407,**
- a sensory component **408,**
- a data transmission component **409.**

The processing component **401** mainly controls all operations of the system **400,** for example, processes data on microbiota, as well as controls the display, phone call, data transmission, camera operation and recording operation of a user's mobile communication device for taking photographs of food products. The processing component **401** may include one or more processors **402** executing commands for the completion of all or some of the stages from the above-mentioned methods. Moreover, the processing component **401** may include one or more modules for convenient interaction between other processing components **401** and other modules. For example, the processing component **401** may include a multimedia module for convenient easier interaction between the multimedia component **405** and the processing component **401.**

The memory **403** is configured to store various types of data to support the operation of the system **400,** such as a database with user profiles. Examples of such data include commands from any application or method, contact details, address book data, messages, images, videos etc., and they all work on the system **400.** The memory **403** can be implemented in the form of any type of volatile memory, non-volatile memory or a combination thereof, including but not limited to static random-access memory (SRAM), electrically erasable programmable read-only memory (EEPROM), erasable programmable read-only memory (EPROM), programmable read-only memory (PROM), read-only memory (ROM), magnetic memory, flash memory, magnetic disk, optical disk etc.

The multimedia component **405** includes a screen providing an output interface between the user and the system **400,** which can be installed on the user's mobile communication device. In some embodiments, the screen can be a liquid crystal display (LCD) or a touch panel (TP). If the screen includes a touch panel, the screen can be implemented as a touch screen to receive input from the user. The touch panel includes one or more touch sensors detecting gestures, touching and sliding of the touch panel. The sensor can sense touching borders or flick gestures, as well as determine the duration of time and pressure associated with the touching and sliding modes. In some embodiments, the multimedia component **405** includes one front-facing camera and/or one rear-facing camera. When the system **400** is in an operating mode, for example, in a photo mode or a video mode, the front-facing camera and/or the rear-facing camera can receive multimedia data externally. Each front-facing camera and rear-facing camera can be one fixed lens optical system or have a focal distance or an optical zoom.

The audio component **406** is configured to output and/or input audio signals. For example, the audio component **406** includes one microphone (MIC) that is configured to receive external audio signals when the system **400** is in an operating mode, for example, in a call mode, a record mode or a voice recognition mode. The audio signal received can further be stored in the memory **403** or can be sent to the data transmission component **409.** In some embodiments, the audio component **406** also includes one loudspeaker configured to output audio signals.

The input/output interface (I/O) **407** provides an interface between the processing component **401** and any peripheral interface module. The above-mentioned peripheral interface module can be a keyboard, a steering wheel, a button etc. These buttons may include a start button, a volume button, an initiate button and a lock button, but this list is non-exhaustive.

The sensory component **408** may include one or more sensors and is configured to provide various aspects of condition assessment of the system **400**. For example, the sensory component **408** can detect on/off conditions of the system **400,** the relative position of its components, such as the display and the keypad, a single component of the system **400,** the presence or absence of contact between the user and the system **400,** as well as the orientation or acceleration/deceleration and temperature change of the system **400**. The sensory component **408** may include a non-contact sensor configured to detect the presence of an object nearby when there is no physical contact. The sensory component **408** may include an optical sensor (for example, a CMOS or a CCD image sensor) configured to be used in the visualization of the application. In some embodiments, the sensory component **408** may include an acceleration sensor, a gyro sensor, a magnetic sensor, a pressure sensor or a temperature sensor.

The data transmission component **409** is configured to facilitate wired or wireless communication between the system **400** and other devices. The system **400** can access a wireless network based on communication standards, such as Wi-Fi, 2G, 3G, 4G, 5G or combinations thereof. In an exemplary embodiment, the data transmission component **409** receives a broadcast signal or a translation, information related to them from an external broadcast control system through a broadcast channel. In one embodiment, the data transmission component **409** may include a near field communication (NFC) module to facilitate near field communication. For example, the NFC module can be based on radio frequency identification (RFID) technology, infrared data association (IrDA) technology, ultra-wideband (UWB) technology, Bluetooth (BT) technology etc.

In an exemplary embodiment, the system **400** can be implemented by means of one or more application-specific integrated circuits (ASIC), a digital signal processor (DSP), digital signal processing devices (DSPDs), a programmable logic device (PLD), a field-programmed gate array (FPGA), a controller, a microcontroller, a microprocessor or other electronic components and can be configured to implement the method 100 of tracking diet and forming conclusions on a user's diet quality and/or personalized nutrition recommendations for a user.

In an exemplary embodiment, a non-volatile computer-readable medium may include the memory **403** that contains commands, where these commands are executed by the processor **401** of the system **400** for implementing the above-mentioned methods of tracking diet and forming conclusions on a user's diet quality and/or personalized nutrition recommendations for a user. For example, the non-volatile computer-readable medium can be read-only memory (ROM), random-access memory (RAM), a compact disk, a magnetic tape, floppy disks, optical storage devices etc.

The computing system **400** may include a display interface that transmits graphics, text and other data from the communication infrastructure (or from a frame buffer, not shown) to display on the multimedia component **405**. The computing system **400** additionally includes input devices or peripheral devices. The peripheral devices may include one or more devices to interact with the user's mobile communication device, such as a keyboard, a microphone, a wearable device, a camera, one or more audio speakers, and other sensors. The peripheral devices can be external or internal to the user's mobile communication device. The touch screen can usually display graphics and text and also provides a user interface (for example, but not limited to a graphical user interface (GUI)) through which the user can interact with the user's mobile communication device, for example, access and interact with applications running on this device, refer to scores of food products and formed nutrition recommendations in detail.

The elements of the claimed invention are in functional interrelationships, and their shared use leads to the creation of a new and unique technical solution. Thus, all the units are functionally related.

All the units used in the system can be implemented using electronic components used to create digital integrated circuits, that is obvious to a person skilled in the art. Chips, the logic of which is specified during manufacturing, or field-programmable gate arrays (FPGAs), the logic of which is specified by programming, can also be used without limitation. For programming, programmers and debugging environments are used that allow you to set the desired structure of a digital device in the form of an electrical schematic diagram or a program in special hardware description languages: Verilog, VHDL, AHDL and others. An alternative to FPGAs can be programmable logic controllers (PLC), master slice arrays (MSA), which require a factory production process for programming, ASIC, i.e. application-specific custom-designed large-scale integrated circuits (LSIC), which are much more expensive for small-batch and individual production.

Usually, an FPGA chip itself consists of the following components:

- configurable logic units that implement the required logical function;
- programmable electronic connections between configurable logic units;
- programmable input/output units that provide communication between the external output of the chip and the internal logic.

The units can also be implemented using read-only memory.

Thus, implementation of all used units is achieved by standard means based on the classical principles of implementing the fundamentals of computer engineering.

As will be understood by a person skilled in the art, the aspects of the present disclosure can be implemented in the form of a system, a method, or a computer program product. Accordingly, various aspects of the present disclosure can be implemented solely as hardware, as software (including application software etc.) or as an embodiment combining software and hardware aspects, which in general may be referred to as the "module", "system" or "architecture". Moreover, aspects of the present disclosure can take the form of a computer program product implemented on one or more computer-readable media with a computer-readable program code implemented on them.

Any combination of one or more computer-readable media can also be used. A computer-readable storage medium can be, without limitation, an electronic, magnetic, optical, electromagnetic, infrared or semiconductor system, a device, an apparatus or any suitable combination thereof. More specifically, examples (non-exhaustive list) of computer-readable storage media include: an electrical connection using one or more wires, a portable computer floppy disk, a hard disk, random-access memory (RAM), read-only memory (ROM), erasable programmable read-only memory (EPROM or flash memory), a fiber optic connection, compact disc read-only memory (CD-ROM), an optical storage device, a magnetic storage device or any combination thereof. In the context of this description, a computer-readable storage medium can be any flexible data medium that can contain or store a program for use by the system, device, apparatus, or in conjunction therewith.

The program code embedded in the computer-readable medium can be transmitted by any medium, including, without limitation, wireless, wired, fiber optic, infrared and any other suitable network or a combination thereof.

The computer program code for performing operations for the stages of the present disclosure can be written in any programming language or a combination of programming languages, including object-oriented programming languages, such as Python, R, Java, Smalltalk, C++ etc., and conventional procedural programming languages, such as the programming language "C" or similar programming languages. The program code can be executed on the user's computer in full, in part, or as a separate software package, partly on the user's computer and partly on the remote computer, or completely on the remote computer. In the latter case, the remote computer may be connected to the user's computer via any type network, including a local area network (LAN), a wide area network (WAN) or a connection to an external computer (for example, via the Internet through internet-service providers).

The aspects of the present disclosure have been described in detail with reference to block diagrams, circuit diagrams and/or diagrams of methods, devices (systems) and computer program products in accordance with embodiments of the present disclosure. It should be noted that each component of the flow chart and/or diagrams, as well as combinations of components of the flow chart and/or diagrams, can be implemented with computer program commands. These computer program commands may be provided to a general-purpose computer processor, a special-purpose computer processor, or another data-processing device to create a procedure in such a way that the commands executed by the computer processor or other programmable data-processing device create the means for implementing the functions/actions specified in a component or components of the flow chart and/or the diagram.

These computer program commands may also be stored on a computer-readable medium, that can control a computer other than a programmable data-processing device or other than devices that operate in a specific manner, in such a way that the commands stored on the computer-readable medium create a device, including commands that perform the functions/actions specified in a component of the flow chart and/or the diagram.

**Claims**

1. A method of tracking diet and forming conclusions on a user's diet quality and/or personalized nutrition recommendations for a user, executed by at least one processor and comprising the following steps:

   • obtaining data on a user's intestinal microbiota condition, containing at least one biomarker value;
   • determining a value of at least one trait related to the user's intestinal microbiota condition, based on at least one biomarker value obtained;
   • generating a list with at least one food product for the user, based on at least one biomarker value obtained or at least one trait value related to the user's intestinal microbiota condition and determined in the previous step;
   • forming a personalized food product rating from the list generated in the previous step;
   • obtaining data on at least one food product or dish of the user;
   • determining a score for at least one food product or dish obtained in the previous step, based on the personalized food product rating formed earlier;
   • forming at least one conclusion on the user's diet quality, based on the score previously determined, and/or at least one recommendation for the user based on the personalized food product rating.

2. The method of claim 1, wherein data on the user's intestinal microbiota condition are obtained by sequencing a biomaterial sample and/or because of a biochemical or microbiological analysis.

3. The method of claim 1, wherein the biomarker is the relative or absolute abundance of microorganisms and/or their genes in the biological material.

4. The method of claim 1, wherein in forming the personalized food product rating, the rating value is calculated from the cumulative effect of this product on each trait associated with the user's intestinal microbiota condition; and the higher is the rating, the more useful is the product for the user's intestinal microbiota.

5. The method of claim 1, wherein the biomarker is positively or negatively associated with a particular trait, depending on the biomarker effect on the trait value, and wherein a food product is positively or negatively associated with a particular biomarker, depending on the food product effect on the biomarker value.

6. The method of claim 1, wherein the trait related to the user's intestinal microbiota condition is the microbiota ability to synthesize metabolites by using the known metabolic pathways, or the microbiota ability to break down dietary fibers, or the glycemic response to a product or a dish, or the ability to break down gluten, or the ability to break down lactose, or microbiota barrier functions, or a disease protection for which the connection with at least one microbiota biomarker or the probiotic level or another beneficial bacteria level is known.

7. The method of claim 1, wherein each trait is evaluated individually or grouped by a trait type with evaluating the entire group of traits in total.

8. The method of claim 1, wherein the initial level of the abundance of a biomarker in the user's intestinal microbiota, or the significance of the trait itself, or the number of biomarkers within one trait or group of traits influenced by a certain product are taken into account when forming the personalized food product rating.

9. The method of claim 1, wherein the rating for a food product is calculated as a weighted sum for all the user's trait when determining a personalized food product rating.

10. The method of claim 1, wherein a shortlist of recommended food products for a predetermined period of time is generated when forming the personalized food product rating.

11. The method of claim 10, wherein the shortlist of recommended food products is randomly generated from a complete list of food products that are positively recommended to the user, where the higher is the probability of a product getting shortlisted for a predetermined period of time, the higher is the rating of this product.

12. The method of claim 1, wherein data on at least one food product or dish of the user is a list of food products or dish ingredients provided by the user, or an image of a dish or a food product, or an audio recording with a list of food products, or the composition of a dish and its cooking method.

13. The method of claim 12, wherein an image of a dish or a food product is recognized by an artificial neural network which receives an input of an image of a dish or a food product and generates an output of a N-dimensional vector of visual features of the image, and the visual information of the image analyzed is encoded in the vector.

14. A system for tracking diet and forming conclusions on a user's diet quality and/or personalized nutrition recommendations for a user, comprising at least one processor and at least one memory connected to the processor and containing a recorded program for carrying out the steps of the method according to claim 1.

15. A computer-readable storage medium containing a program for automating the method of tracking diet and forming conclusions on a user's diet quality and/or personalized nutrition recommendations for a user, wherein the program contains:

a. commands for obtaining data on a user's intestinal microbiota condition, containing at least one biomarker value;
b. commands for determining a value of at least one trait related to the user's intestinal microbiota condition based on at least one biomarker value obtained;
c. commands for generating a list with at least one food product for the user, based on at least one biomarker value obtained or at least one trait value related to the user's intestinal microbiota condition and determined in the previous step;
d. commands for forming a personalized food product rating from the list generated in the previous step;
e. commands for obtaining data on at least one food product or dish of the user;

f. commands for determining a score for at least one food product or dish obtained in the previous step, based on the personalized food product rating formed earlier;

g. commands for forming conclusions on the user's diet quality, based on the score previously determined, and/or at least one recommendation for the user based on the personalized food product rating.

~ 100

110 Obtaining data on a user's intestinal microbiota condition, containing at least one biomarker value

120 Determining a value of at least one trait related to the user's intestinal microbiota condition, based on at least one biomarker value obtained

130 Generating a list with at least one food product for the user, based on at least one biomarker value obtained and/or at least one trait value related to the user's microbiota condition and determined in the previous step

140 Forming a personalized food product rating from the list generated in the previous step

150 Obtaining data on at least one food product or dish of the user

160 Determining a score for at least one food product or dish obtained in the previous step, based on the food product rating formed earlier

170 Forming a conclusion on the user's diet quality, based on the score previously obtained, and/or at least one recommendation for the user, based on the personalized food product rating

Fig. 1

Fig. 2

Fig. 3

Fig. 4

500

**501** A list of positively recommended food products

**502** A change to the product rating values through ranking and/or predetermined factors

**503** Calculation of the ratio of food products from each category to the total number of positively recommended food products

**504** Determination of the number of places for each food category in a list for a week, based on the ratio obtained

**505** Calculation of the probability of a food product getting into a new list for a week, based on the changed rating values

**506** Random selection of food products from each category of the list of recommended food products according to the resulting number of places

**507** A shortened list of recommended food products for a predetermined period of time

Fig. 5

## INTERNATIONAL SEARCH REPORT

| | International application No. |
|---|---|
| | PCT/RU 2020/050312 |

| A. CLASSIFICATION OF SUBJECT MATTER | |
|---|---|
| | *G16H 20/60 (2018.01)* |
| | *G06Q 90/00 (2006.01)* |

According to International Patent Classification (IPC) or to both national classification and IPC

| B. FIELDS SEARCHED |
|---|
| Minimum documentation searched (classification system followed by classification symbols) |
| G06F 3/00, 17/00, 17/40, G06Q 50/00, 50/10, 50/22, H04L 29/00, 29/02, 29/04, 29/06, 29/08 |

Documentation searched other than minimum documentation to the extent that such documents are included in the fields searched

Electronic data base consulted during the international search (name of data base and, where practicable, search terms used)

PatSearch (RUPTO internal), USPTO, PAJ, Esp@cenet, DWPI, EAPATIS, PATENTSCOPE, Information Retrieval System of FIPS

| C. DOCUMENTS CONSIDERED TO BE RELEVANT | | |
|---|---|---|
| Category* | Citation of document, with indication, where appropriate, of the relevant passages | Relevant to claim No. |
| A | RU 2639269 C2 (KORSAKOV DMITRY BORISOVICH et al.) 20.12.2017 | 1-15 |
| A | US 2016/0307128 A1 (TUTSHO, EEC) 20.10.2016 | 1-15 |
| A | US 2017/0018199 A1 (GENES ANT TECHNOLOGIES, INC.) 19.01.2017 | 1-15 |
| A | US 8690578 B1 (MARK E. NUSBAUM et al.) 08.04.2014 | 1-15 |
| A | US 2016/0063734 A1 (SRI INTERNATIONAL) 03.03.2016 | 1-15 |

☐ Further documents are listed in the continuation of Box C.     ☐ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 March 2021 (16.03.2021) | 01 April 2021 (01.04.2021) |

| Name and mailing address of the ISA/    RU | Authorized officer |
|---|---|
| | |
| Facsimile No. | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KLIMENKO N. S. et al.** Microbiome Responses to an Uncontrolled Short-Term Diet Intervention in the Frame of the Citizen Science. *Project. Nutrients.,* 08 May 2018, vol. 10 (5